# EUROPÄISCHE PATENTSCHRIFT

(11) **EP 0 583 324 B1**
(45) Veröffentlichungstag und Bekanntmachung des Hinweises auf die Patenterteilung: **21.12.1994**
(21) Anmeldenummer: 92909829.1
(22) Anmeldetag: 07.05.1992
(51) Int. Cl.: A61K 7/13

(54) **HAARFÄRBEMITTEL**
HAIR-DYEING AGENT
AGENTS DE TEINTURE POUR CHEVEUX

(30) Priorität: 16.05.1991 DE 4115983
(43) Veröffentlichungstag der Anmeldung: 23.02.1994
(73) Patentinhaber: Henkel Kommanditgesellschaft auf Aktien, 40191 Düsseldorf (DE)
(72) Erfinder: ROSE, David, D-4010 Hilden (DE); LIESKE, Edgar, D-4000 Düsseldorf (DE); HÖFFKES, Horst, D-4000 Düsseldorf 13 (DE)
(86) Internationale Anmeldenummer: EP9200999
(87) Internationale Veröffentlichungsnummer: WO9220320

(56) Entgegenhaltungen:
- EP-A- 0 170 069
- FR-A- 1 508 405
- GB-A- 1 116 138
- US-A- 2 687 431

## Beschreibung

Die Erfindung betrifft Färbemittel für Keratinfasern, insbesondere für menschliche Haare. Solche Färbemittel enthalten direktziehende Haarfarbstoffe in einem kosmetischen Träger. Als kosmetische Träger dienen meist wäßrige Zubereitungen, z. B. in Form von Cremes, Emulsionen, Gelen, Shampoos, Schaumaerosole oder anderen Zubereitungen, die für die Anwendung auf dem Haar geeignet sind.

Für das Färben von Haar spielen neben den Oxidationsfarben, besonders die direktziehenden Haarfarbstoffe eine wichtige Rolle. Die direktziehenden Farbstoffe haben den Vorteil, daß sie ohne Zusatz von Oxidationsmitteln zur Anwendung kommen. Als direktziehende Farbstoffe werden vorwiegend Verbindungen eingesetzt, die zur Gruppe der Nitrobenzolderivate gehören. Sie werden entweder allein oder in Kombination mit anderen direktziehenden Farbstoffen wie Anthrachinonfarbstoffen, Indophenolen oder mit Oxidationsfarbstoffen eingesetzt.

Gute Haarfärbemittel müssen die gewünschten Farbnuancen in ausreichender Intensität ausbilden. Sie müssen ein gutes Aufziehvermögen auf menschlichem Haar besitzen ohne die Kopfhaut zu stark anzufärben. Die damit erzeugten Färbungen müssen eine hohe Stabilität gegen Licht, Wärme, Schweiß, Haarwaschmittel und die bei der Dauerwellung des Haares verwendeten Chemikalien aufweisen. Sie sollen schließlich in toxikologischer und dermatologischer Hinsicht unbedenklich sein.

Unter den direktziehenden Nitrobenzolderivaten spielen die Nitroaniline und deren Derivate eine besondere Rolle, da einige dieser Farbstoffe intensive Färbungen ausbilden. Ein Nachteil der bekannten direktziehenden Nitroanilin-Farbstoffe sind jedoch eine unbefriedigende Echtheitseigenschaften, d. h. daß die Haarfärbungen durch das Haarewaschen oder durch das Sonnenlicht aufgehellt und in ihrer Farbnuance verändert werden.

Römpps Chemie Lexikon (8. Auflage 1985, 2821) beschreibt, daß Nitrofarbstoffe mit saurem Charakter, die neben Nitrogruppen noch o- und p-ständige Amino- bzw. Hydroxygruppen enthalten, schlechte Waschechtheit aufweisen.

So sind z. B. aus DE 34 25 151 A1 Amino-nitrobenzoesäuren und Amino-nitrobenzolsulfonsäuren bekannt, welche die an die Lichtechtheit gestellten Anforderungen noch nicht voll befriedigen.

Direktziehende Farbstoffe sollen darüber hinaus eine gute Verträglichkeit mit anderen Farbstoffen, z. B. mit Oxidationsfarbstoffvorprodukten und mit den in Oxidationshaarfärbemitteln üblichen Komponenten aufweisen, da man zur Nuancenabwandlung gerne direktziehende Farbstoffe und Oxidationsfarbstoffe kombiniert. Daher ist eine gute Stabilität gegen Reduktionsmittel und gegen Oxidationsmittel erforderlich.

Es wurde nun gefunden, daß Haarfärbemittel mit einem Gehalt an direktziehenden Nitrofarbstoffen in einem wäßrigen Träger die gestellten Anforderungen in einem hohen Maße erfüllen, wenn als direktziehender Nitro-farbstoff wenigstens eine Verbindung der Formel I 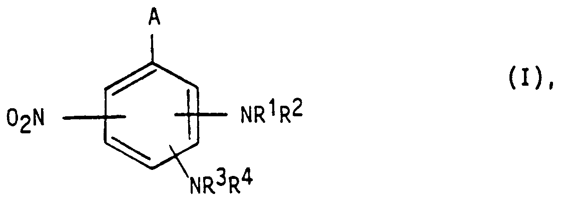
in der A eine SO₃H-Gruppe oder eine COOH-Gruppe, R¹, R², R³ und R⁴ Wasserstoff, Alkylgruppen mit 1 - 4 C-Atomen, Hydroxyalkylgruppen mit 2 - 4 C-Atomen darstellen oder R¹ und R² sowie R³ und R⁴ jeweils gemeinsam mit dem Stickstoffatom einen Piperidin-, Morpholin- oder Piperazinring bilden und worin die NO₂-Gruppe und die Gruppen -NR¹R² und -NR³R⁴ beliebige Positionen am Ring einnehmen können, oder deren wasserlösliches Salz enthalten ist.

Die Farbstoffe der Formel I erzeugen auf Keratinfasern gelbe bis braunorange Farbnuancen hoher Intensität und guter Echtheitseigenschaften. Sie besitzen im Vergleich zu Amino-nitro-benzolsulfonsäuren und -benzoesäuren mit nur einer Aminogruppe bessere Lichtechtheitseigenschaften. In dermatologischer und toxikologischer Hinsicht sind die Verbindungen unschädlich.

Viele Verbindungen der Formel I sind literaturbekannt. Solche Verbindungen der Formel I, die nicht literaturbekannt sind, lassen sich nach bekannten Syntheseverfahren herstellen. Beispiele für solche Verbindungen sind unten (Beispiel 1.1 bis 1.4) angegeben.

Bevorzugt, auch wegen ihrer besseren Zugänglichkeit, werden als direktziehende Nitro-Haarfarbstoffe solche der Formel Ia 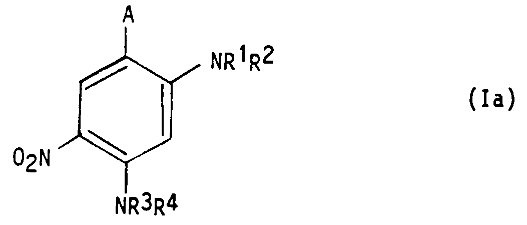
eingesetzt, worin A, R¹, R², R³ und R⁴ die für Formel I angegebene Bedeutung haben. Weiterhin sind solche Farbstoffe der Formel I oder Ia bevorzugt, worin R¹, R², R³ und R⁴ Wasserstoff, eine Methylgruppe oder eine Hydroxyethylgruppe sind oder die Gruppen R¹ und R² oder R³ und R⁴ jeweils gemeinsam mit dem Stickstoffatom einen Morpholin- oder Piperidinring bilden.

Geeignete Verbindungen der allgemeinen Formel I, in der A eine -COOH-Gruppe darstellt, sind z. B.
3-Amino-4-methylamino-5-nitrobenzoesäure
2-4-Bis-(2-hydroxyethyl)-amino-5-nitrobenzoesäure
2,4-Bis-morpholino-5-nitro-benzoesäure
2,4-Bis-piperidino-5-nitrobenzoesäure
2-Nitro-3,5-diaminobenzoesäure
2,3-Diamino-5-nitro-benzoesäure,
2,4-Diamino-5-nitro-benzoesäure und
3,4-Diamino-5-nitro-benzoesäure.

Geeignete Verbindungen der Formel I, in der A eine -SO₃H-Gruppe darstellt, sind z. B.
2,4-Bis-dimethylamino-5-nitro-benzolsulfonsäure
2,4-Diamino-5-nitro-benzolsulfonsäure und
3,4-Diamino-5-nitro-benzolsulfonsäure.

Die erfindungsgemäßen Haarfärbemittel können die direktziehenden Nitroanilinderivate der allgemeinen Formel I allein oder in Kombination mit bekannten direktziehenden Farbstoffen, z. B. mit anderen Nitrobenzolderivaten, Anthrachinonfarbstoffen oder Azofarbstoffen enthalten. Darüber hinaus eignen sich die direktziehenden Farbstoffe der allgemeinen Formel I wegen ihrer hohen Beständigkeit gegenüber Reduktionsmitteln und Oxidationsmitteln auch sehr gut zur Kombination mit Oxidationsfarbstoffvorprodukten, also zur Modifikation der Nuancen von Oxidationshaarfärbemitteln. Oxidationshaarfärbemittel enthalten als Farbstoffvorprodukte Entwicklerkomponenten, die durch oxidative Kupplung untereinander oder mit geeigneten Kupplerkomponenten die Oxidationsfarbstoffe ausbilden. Als Entwicklersubstanzen werden z. B. primäre aromatische Amine mit einer weiteren, in Para- oder Orthoposition befindlichen freien oder substituierten Hydroxy- oder Aminogruppe, Diaminopyridinderivate, heterocyclische Hydrazone, 4-Aminopyrazolonderivate und 2.4.5.6-Tetraaminopyrimidin und dessen Derivate eingesetzt. Als Kupplersubstanzen werden m-Phenylendiaminderivate, Naphthole, Resorcin und Resorcinderivate, Pyrazolone und m-Aminophenole verwendet.

Zur Herstellung der erfindungsgemäßen Haarfärbemittel werden die direktziehenden Haarfarbstoffe und gegebenenfalls die Oxidationsfarbstoffvorprodukte in einen geeigneten kosmetischen Träger, z. B. in Cremes, Emulsionen, Gele oder auch tensidhaltige, schäumende Lösungen, z. B. in Shampoos, Schaumaerosole oder andere Zubereitungen eingearbeitet, die für die Anwendung auf dem Haar geeignet sind.

Übliche Bestandteile solcher kosmetischer Zubereitungen sind z. B.:
- Netz- und Emulgiermittel wie anionische, nichtionische oder ampholytische Tenside, z. B. Fettalkoholsulfate, Alkansulfonate, α-Olefinsulfonate, Fettalkoholpolyglycolethersulfate, Ethylenoxidanlagerungsprodukte an Fettalkohole, an Fettsäuren, an Alkylphenole, an Sorbitanfettsäureester, an Fettsäurepartialglyceride und an Fettsäurealkanolamide
- Verdickungsmittel wie z. B. Fettalkohole, Fettsäuren, Paraffinöle, Fettsäureester und andere Fettkomponenten in emulgierter Form,
- wasserlösliche, polymere Verdickungsmittel wie z. B. Methyl- oder Hydroxyethylcellulose, Stärke, Pflanzengumme, wasserlösliche synthetische Polymerisate, wasserlösliche Biopolymere (z. B. Xanthan-Gum)
- haarpflegende Zusätze wie z. B. wasserlösliche, kationische Polymere, Proteinderivate, Pantothensäure, Vitamine, Pflanzenextrakte, Cholesterin und Zucker
- Elektrolyt- und Puffersalze, pH-Stellmittel, Komplexbildner und Parfümöle.

Die Bestandteile der kosmetischen Träger werden zur Herstellung der erfindungsgemäßen Haarfärbemittel in für diese Zwecke üblichen Mengen eingesetzt; z. B. werden Emulgiermittel in Konzentrationen von 0,5 bis 30 Gew.-% und Verdickungsmittel in Konzentrationen von 0,1 bis 25 Gew.-% des gesamten Färbemittels eingesetzt
In den erfindungsgemäßen Haarfärbemitteln werden die direktziehenden Haarfarbstoffe der allgemeinen Formel I in einer Menge von 0,01 bis 5,0 Gew.-%, bevorzugt von 0,1 - 2 Gew.-%, bezogen auf das gesamte Haarfärbemittel, eingesetzt. Daneben können bekannte Oxidationshaarfärbemittelvorprodukte (Entwickler- und Kupplersubstanzen) in einer Menge von 0,1 - 5, vorzugsweise von 1 - 3 Gew. -% enthalten sein.

Wenn das erfindungsgemäße Haarfärbemittel Oxidationsfarbstoffvorprodukte enthält, empfiehlt sich außerdem die Zugabe einer kleinen Menge eines Reduktionsmittels, z. B. von 0,5 - 2,0 Gew.-% Natriumsulfit, zur Stabilisierung der Oxidationsfarbstoffvorprodukte. In diesem Falle wird vor der Anwendung des Haarfärbemittels dieses mit einem Oxidationsmittel versetzt, um die oxidative Entwicklung der Oxidationsfarbstoffvorprodukte einzuleiten. Als Oxidationsmittel kommen insbesondere Wasserstoffperoxid oder dessen Anlagerungsprodukte an Harnstoff, Melamin oder Natriumborat sowie Gemische aus derartigen Wasserstoffperoxidanlagerungsprodukten mit Kaliumperoxidisulfat in Frage. Die Anwendung der erfindungsgemäßen Haarfärbemittel kann, unabhängig von der Art der kosmetischen Zubereitung, zum Beispiel als Creme, Gel oder Shampoo, im schwach sauren, neutralen oder alkalischen Milieu erfolgen, bevorzugt ist die Anwendung der Haarfärbemittel in einm pH-Bereich von 8 - 10. Die Anwendungstemperaturen können in einem Bereich zwischen 15°C und 40°C liegen. Nach einer Einwirkungszeit von ca. 30 Minuten wird das überschüssige Haarfärbemittel durch Ausspülen von dem zu färbenden Haar entfernt. Danach wird das Haar mit einem milden Shampoo nachgewaschen und getrocknet. Das Nachwaschen mit einem Shampoo entfällt, wenn ein stark tensidhaltiger Träger, z. B. ein Färbeshampoo, verwendet wurde.

Mit den erfindungsgemäßen Haarfärbemitteln lassen sich Haaranfärbungen von hoher Intensität und guten Echtheitseigenschaften, besonders auch von guter Lichtechtheit und hoher Stabilität gegen Ausbluten und Farbveränderungen beim Shamponieren erzielen. Die nachfolgenden Beispiele sollen den Erfindungsgegenstand näher erläutern ohne ihn jedoch hierauf zu beschränken.

### Beispiele

### 1. Herstellungsbeispiele

### 1.1 3-Amino-4-methylamino-5-nitrobenzoesäure

6,7 g 4-Methylamino-3,5-dinitrobenzoesäure (nach J. Chem. Soc. 1951, 2471) wurden in einer Mischung aus 80 ml 1,2-Dimethyoxyethan, 8 ml Chloroform und 30 ml Ethanol gelöst und nach Zugabe von Palladium (auf Aktivkohle) bei 20°C mit Wasserstoff hydriert. Nach der Aufnahme von 2 l Wasserstoff wurde die Hydrierung beendet, der Katalysator durch Filtration entfernt und die Lösung zur Trockene eingedampft. Der Trockenrückstand wurde aus einem Wasser/Ethanol-Gemisch umkristallisiert. Es wurden braune Kristalle mit einem Schmelzpunkt von 224 - 234°C erhalten.

### 1.2 2,4-Bis-(2-hydroxyethyl)-amino-5-nitrobenzoesäure

11,8 g 2,4-Dichlor-5-nitrobenzoesäure (nach Helv. Chim. Acta 40 (1957), 1187) wurden in einer Mischung aus 6,1 g Monoethanolamin, 10,1 g Triethanolamin und 100 ml Wasser gelöst und 24 Stunden lang auf 120 °C erhitzt. Dann wurde das Reaktionsgemisch auf 20°C gekühlt, mit vedünnter Salzsäure angesäuert und der Niederschlag abfiltriert und aus Ethanol umkristallisiert. Es wurden gelbe Kristalle mit einem Schmelzpunkt von 229 - 234°C erhalten.

### 1.3 2,4-Bis-morpholino-5-nitrobenzoesäure

Die Herstellung erfolgte analog Beispiel 2, wobei anstelle von Monoethanolamin eine äquimolare Menge Morpholin eingesetzt wurde. Es wurden orangefarbene Kristalle mit einem Schmelzpunkt von 200 - 207°C erhalten.

### 1.4 2,4-Bis-dimethylamino-5-nitro-benzolsulfonsäure-Kaliumsalz

10 g 2,4-Dichlor-5-nitro-benzolsulfonsäure-K-Salz (nach DD 120 345) wurden in einer Lösung von 20 g Dimethylamin in 30 g Wasser gelöst und nach Zugabe von 2 g CuCl 24 Stunden auf 120°C erhitzt. Nach dem Abkühlen auf 20°C wurde das Reaktionsgemisch filtriert und das Filtrat zur Trockene eingedampft. Der Rückstand wurde aus Wasser umkristallisiert. Es wurden gelbe Kristalle mit einem Schmelzpunkt von 210°C (unter Zersetzung) erhalten.

Folgende weitere Verbindungen wurden nach Literaturangaben hergestellt.
1.5 2,4-Diamino-5-nitro-benzolsulfonsäure (nach DD 120 345)
1.6 2,4-Bis-piperidino-5-nitro-benzoesäure (nach EP 23 569)
1.7 2-Nitro-3,5-diaminobenzoesäure (nach Zh. Org. Khim. 10 (10) (1974), 2187)
1.8 2,3-Diamino-5-nitro-benzoesäure (nach J. Org. Chem. (1960), 943)

### 2. Haarfärbversuche

Es wurden Haarfärbecremes der folgenden Zusammensetzung hergestellt:

| | |
|---|---|
| Fettalkohol C₁₂₋₁₈ | 10 g |
| Fettalkohol C₁₂₋₁₄+2 EO-sulfat, Na-Salz (28%ig) | 25 g |
| Wasser | 60g |
| direktziehender Farbstoff | 1 g |
| Ammoniumsulfat | 1 g |
| konzentrierte Ammoniak-Lösung | bis pH=9,5 |
| Wasser | ad 100 g |

Die Bestandteile wurden der Reihe nach miteinander vermischt. Nach Zugabe der direktziehenden Farbstoffe wurde mit konzentrierter Ammoniak-Lösung der pH-Wert der Emulsion auf 9,5 eingestellt, dann wurde mit Wasser auf 100 g aufgefüllt
Die Färbecreme wurde auf ca. 5 cm lange Strähnen standardisierten, zu 90 % ergrauten, aber nicht besonders vorbehandelten Menschenhaars aufgetragen und dort 30 Minuten bei 27°C belassen. Nach Beendigung des Färbeprozesses wurde das Haar gespült, mit einem üblichen Haarwaschmittel ausgewaschen und anschließend getrocknet.

Als direktziehende Haarfarbstoffe wurden die Verbindungen gemäß Beispiel 1.1 bis 1.8 eingesetzt. Es wurden die folgenden Haaranfärbungen erhalten:

| Beispiel | eingesetzter Farbstoff | Nuance des gefärbten Haares |
|---|---|---|
| 2.1 | 1.1 | gelborange |
| 2.2 | 1.2 | olivgelb |
| 2.3 | 1.3 | absinthgelb |
| 2.4 | 1.4 | graugelb |
| 2.5 | 1.5 | olivbraun |
| 2.6 | 1.6 | olivgelb |
| 2.7 | 1.7 | lindengrün |
| 2.8 | 1.8 | braunorange |

## Patentansprüche

1. Haarfärbemittel mit einem Gehalt an direktziehenden Nitro-farbstoffen in einem wäßrigen Träger, dadurch gekennzeichnet, daß als direktziehender Nitro-farbstoff wenigstens eine Verbindung der Formel I 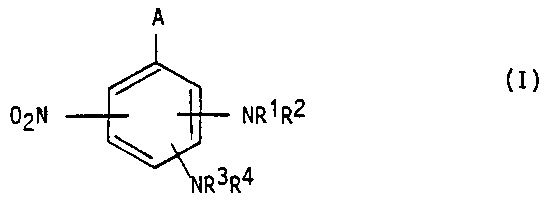 in der A eine SO₃H-Gruppe oder eine COOH-Gruppe, R¹, R², R³ und R⁴ Wasserstoff, Alkylgruppen mit 1 - 4 C-Atomen, Hydroxyalkylgruppen mit 2 - 4 C-Atomen darstellen oder R¹ und R² sowie R³ und R⁴ jeweils gemeinsam mit dem Stickstoffatom eine Piperidin-, Morpholin- oder Piperazinring bilden und worin die NO₂-Gruppen und die Gruppen -NR¹R² und -NR³R⁴ beliebige Positionen am Ring einnehmen können, oder deren wasserlösliches Salz enthalten ist.

2. Haarfärbemittel nach Anspruch 1, dadurch gekennzeichnet, daß als direktziehender Nitro-farbstoff wenigstens eine Verbindung der Formel Ia 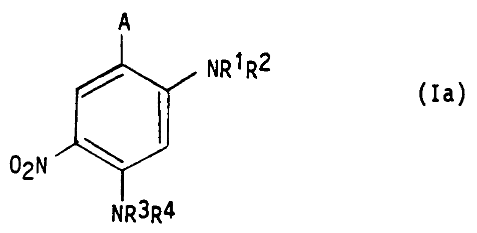 enthalten ist, in der A, R¹, R², R³ und R⁴ die in Anspruch 1 angegebene Bedeutung haben.

3. Haarfärbemittel nach Anspruch 1 oder 2, dadurch gekennzeichnet, daß R¹, R², R³ und R⁴ Wasserstoff, eine Methylgruppe, eine Hydroxyethylgruppe sind oder die Gruppen R¹ und R² oder R³ und R⁴ gemeinsam mit dem Stickstoffatom einen Morpholin- oder Piperidinring bilden.

4. Haarfärbemittel nach einem der Ansprüche 1 - 3, dadurch gekennzeichnet, daß die Verbindungen der Formel I oder Ia in einer Menge von 0,01 bis 5 Gew.-% bezogen auf das gesamte Haarfärbemittel enthalten sind.

## Claims

1. Hair dyes containing substantive nitro dyes in an aqueous carrier, characterized in that at least one compound corresponding to formula I: 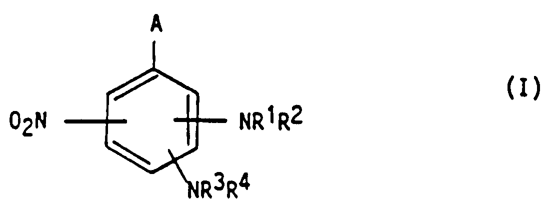 in which A is an SO₃H group or a COOH group, R¹, R², R³ and R⁴ represent hydrogen, C₁₋₄ alkyl groups, C₂₋₄ hydroxyalkyl groups or R¹ and R² and also R³ and R⁴ together with the nitrogen atom form a piperidine, morpholine or piperazine ring and in which the NO₂ groups and the -NR¹R² and -NR³R⁴ groups may occupy any positions on the ring, or a water-soluble salt thereof is present as the substantive nitro dye.

2. Hair dyes as claimed in claim 1, characterized in that at least one compound corresponding to formula Ia: 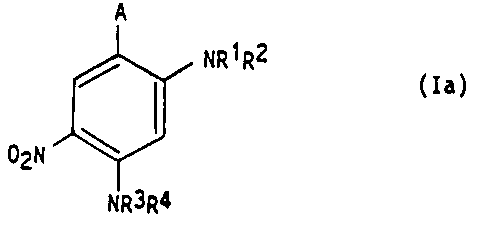 in which A, R¹, R², R³ and R⁴ are as defined in claim 1, is present at the substantive nitro dye.

3. Hair dyes as claimed in claim 1 or 2, characterized in that R¹, R², R³ and R⁴ represent hydrogen, a methyl group or a hydroxyethyl group or the groups R¹ and R² or R³ and R⁴ together with the nitrogen atom form a morpholine or piperidine ring.

4. Hair dyes as claimed in any of claims 1 to 3, characterized in that the compounds corresponding to formula I or Ia are present in a quantity of 0.01 to 5% by weight, based on the hair dye as a whole.

## Revendications

1. Agents de teinture pour cheveux avec une teneur en nitro-colorants montant directement sur le cheveu dans un support aqueux, caractérisés en ce que comme nitro-colorant est contenu au moins un composé de formule (I) ou un de leurs sels solubles dans l'eau : 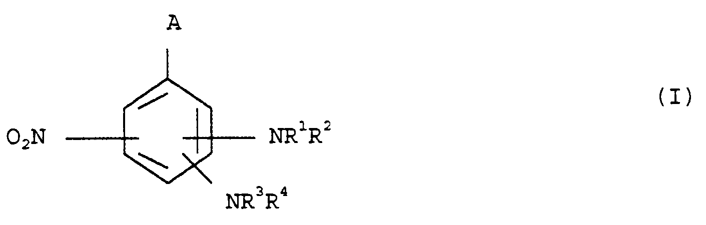 dans laquelle A représente un groupe SO₃H ou un groupe -COOH, R¹, R, R³ et R⁴ de l'hydrogène, des groupes alkyle avec 1-4 atomes de C, des groupes hydroalkyle avec 2-4 atomes de C ou bien R¹ et R² ainsi que R³ et R⁴ forment respectivement en commun avec l'atome d'azote un composé cyclique de pipéridine, de morpholine, ou de pipérazine et dans laquelle le groupe NO₂ et les groupes -NR¹R² et -NR³R⁴ peuvent prendre des positions quelconques sur le cycle.

2. Agents de teinture pour cheveux selon la revendication 1, caractérisés en ce que comme nitro-colorant montant directement est contenu au moins un composé de formule (Ia) : 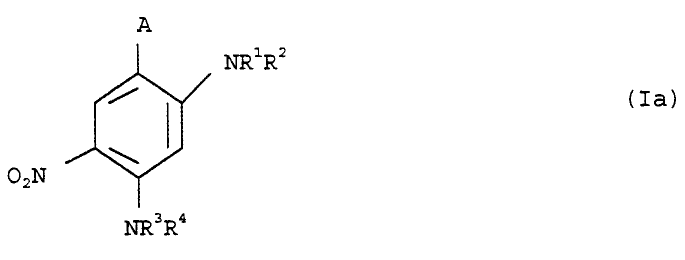 dans laquelle A, R₁, R₂, R₃ et R₄ ont la signification indiquée à la revendication 1.

3. Agents de teinture pour cheveux selon les revendications 1 ou 2, caractérisés en ce que R¹, R², R³ et R⁴ sont de l'hydrogène, un groupe méthyle, un groupe hydroxyéthyle ou bien les groupes R¹ et R² ou R³ et R⁴ forment en commun avec l'atome d'azote un composé cyclique de morpholine ou de pipéridine.

4. Agents de teinture pour cheveux selon une des revendications 1 à 3, caractérisés en ce que les composés de formule (I) ou (Ia) sont contenus en une quantité de 0,01 à 5 % en poids, rapportée à la totalité de l'agent de teinture.
